# EUROPEAN PATENT SPECIFICATION

(11) **EP 1 072 579 B1**
(45) Date of publication and mention of the grant of the patent: **06.05.2004**
(21) Application number: 00115057.2
(22) Date of filing: 26.07.2000
(51) Int. Cl.: C07C 51/15, C07C 65/03, C07C 65/05, C07C 65/11, C07C 227/02

(54) **Method for producing aromatic carboxylic acid compounds**
Verfahren zur Herstellung von aromatischen Carbonsäuren
Procédé de préparation de composés d'acides carboxyliques aromatiques

(30) Priority: 30.07.1999 JP 21697399; 28.09.1999 JP 27502199; 27.03.2000 JP 2000087557
(43) Date of publication of application: 31.01.2001
(73) Proprietor: FUJI PHOTO FILM CO., LTD., Kanagawa 250-01 (JP)
(72) Inventor: Takaku, Koji, Minami-Ashigara-shi, Kanagawa-ken (JP); Ito, Takayuki, Minami-Ashigara-shi, Kanagawa-ken (JP)
(74) Representative: HOFFMANN - EITLE

(56) References cited:
- EP-A- 0 552 019
- EP-A- 0 834 494
- WO-A-97/44305
- US-A- 3 532 745
- US-A- 3 825 593
- US-A- 4 032 555

## Description

### FIELD OF THE INVENTION

The present invention relates to an improved production method of phenols and naphthols, each having a carboxyl group introduced at the 2- or 4-position thereof.

### BACKGROUND OF THE INVENTION

The Kolbe-Schmitt reaction, which consists of reacting phenols or naphthols with carbon dioxide in the presence of an alkali metal salt, or alternatively reacting an alkali metal salt of phenols or naphthols with carbon dioxide, at a high temperature, while applying high pressure, thereby introducing a carboxyl group at the 2-or 4-position thereof, is a reaction that can be used for a wide variety of industrial purposes. However, there is a problem that the reaction is so dependent on the kind of substrates, that some substrates need both high temperature and pressure conditions. Further, according to Chem. Rev. 57, 583 (1957), a method of performing the reaction can be classified into two kinds, namely a heterogeneous reaction between vapor phase/solid phase, and a homogeneous reaction between vapor phase/liquid phase. In the vapor phase/solid phase method, there is a problem that reliance on apparatuses for the manufacturing is so high, and the reaction becomes complicated. On the other hand, the vapor phase/liquid phase method has an advantage that such reliance on apparatuses is low. However, with the vapor phase/liquid phase method, there is another problem, that the reaction stops as a result of the effect of water that formed at the time of formation of alkali metal salt of phenols or naphthols. In each of Journal of Synthetic Organic Chemistry, Japan (Yuki Goseikagaku Kyokai Shi), 326 (1976), JP-A-58-99436 (the term "JP-A" as used herein means an "unexamined published Japanese patent application"), and JP-A-1-180863, there is therefore described a method in which alkali metal salts of phenols or naphthols are previously prepared, they are isolated and dried up by evaporation, and then a reaction is conducted using the dried-up alkali metal salts in accordance with the vapor phase/liquid phase method. Further, European patent No. 927153 describes a method in which alkali metal salts of phenols or naphthols are previously prepared in an organic solvent that can form an azeotropic mixture with water, then water is eliminated from the mixture, and thereafter, without isolating the alkali metal salts, a reaction is conducted in accordance with the vapor phase/liquid phase method. Further, as a method of using a strong base that does not form water at the time of the alkali metal salt-making, JP-A-5-194314 (corresponding publication: EP-A-552,019) describes a method in which a dihydroxynaphthalene is employed as a starting raw material, and its alkaline metal salt is prepared by using an alkali metal alkoxide in an organic solvent, then the formed alcohol is eliminated by heating, and thereafter a reaction is conducted in accordance with the vapor phase/liquid phase method. However, it is difficult to say that these methods are industrially advantageous, because the method is composed of many steps, and consequently operations also become complicated because, after isolating the alkali metal salt, the isolated salt and carbon dioxide are reacted, or because the formed water or alcohol is eliminated by azeotropic distillation or so on. Further, in the foregoing methods (Journal of Synthetic Organic Chemistry of Japan, JP-A-58-99436, and JP-A-1-180863), a problem arises that a yield obtained by unstable compounds such as those capable of being oxidized, or compounds exhibiting a high melting point, is low.

### SUMMARY OF THE INVENTION

An object of the present invention is to provide a method for introducing a carboxyl group at the 2- or 4-position of phenols or naphthols, under a relatively moderate reaction condition and in a manner advantageous to industrial production.

Other and further objects, features, and advantages of the invention will appear more fully from the following description.

### DETAILED DESCRIPTION OF THE INVENTION

As a result of an intensive investigation, the present inventors have found a method comprising using sodium t-butoxide as a strong base, whereby a reaction can be performed in a high yield and free from both isolation of an alkali metal salt and operation of eliminating water, alcohol or the like. More minutely, a carboxyl group can be introduced at the 2- or 4-position of phenols and naphthols under a relatively moderate reaction condition and in the manner advantageous to the industrial production, by preparing an alkali metal salt of the phenols and the naphthols by the use of sodium t-butoxide, in an aprotic (a non-protic) polar solvent, and then conducting a reaction of the alkali metal salt and carbon dioxide without conducting both isolation of the alkali metal salt and operation to eliminate the formed alcohol.

That is, an object of the present invention has been accomplished by the following (1) and (2).
(1) A method for producing a compound represented by formula (II), comprising reacting a compound represented by formula (I) and carbon dioxide, in the presence of sodium t-butoxide in an aprotic polar solvent, thereby introducing a carboxyl group at the 2- or 4-position thereof: 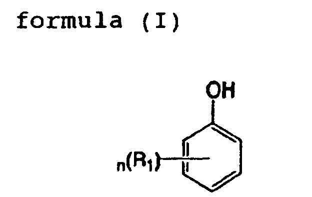 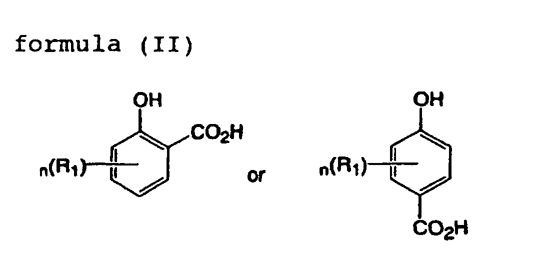 wherein R₁ represents a substituent, with a proviso that any one of R₁'s at ortho or para position to the hydroxyl group is a hydrogen atom; n represents an integer of 0 to 4; when n is 2 or greater, a plurality of R₁'s may be the same or different, and R₁'s positioned ortho with each other, may be combined to form an aliphatic or aromatic 5-to 7-membered ring.
(2) A method for producing a compound represented by formula (IV), comprising reacting a compound represented by formula (III) and carbon dioxide, in the presence of sodium t-butoxide in an aprotic polar solvent, thereby introducing a carboxyl group at the 2-position thereof: 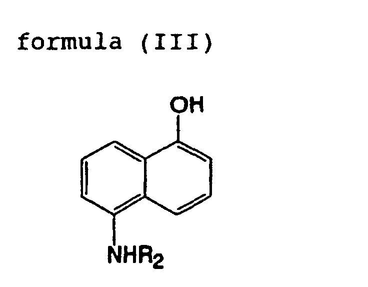 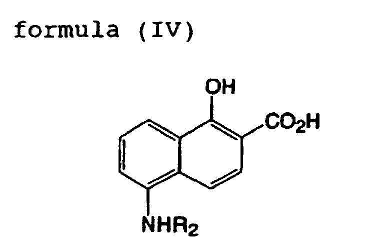 wherein R₂ represents a hydrogen atom, an alkyl group, an acyl group, an alkylsulfonyl group, an arylsulfonyl group, an alkoxycarbonyl group, an alkylcarbamoyl group, or an arylcarbamoyl group.

The present invention is explained below in detail.

In formula, R₁ represents a hydrogen atom, a halogen atom, an alkyl group (the term "alkyl group" in this specification includes a cycloalkyl group, and a bicycloalkyl group), an alkenyl group (the term "alkenyl group" in this specification includes a cycloalkenyl group, and a bicycloalkenyl group), an alkynyl group, an aryl group, a heterocyclic group, a cyano group, a hydroxyl group, a nitro group, a carboxyl group, an alkoxy group, an aryloxy group, a heterocyclicoxy group, an acyloxy group, an amino group (the term "amino group" in this specification includes an anilino group), an acylamino group, an aminocarbonylamino group, an alkoxycarbonylamino group, a sulfamoylamino group, an alkyl- or aryl- sulfonylamino group, a mercapto group, an alkylthio group, an arylthio group, a heterocyclicthio group, a sulfamoyl group, a sulfo group, an alkyl- or aryl-sulfinyl group, an alkyl- or aryl-sulfonyl group, an acyl group, an aryloxycarbonyl group, an alkoxycarbonyl group, a carbamoyl group, an imido group, a phosphino group, a phosphinyl group, a phosphinylamino group, or a silyl group, with a proviso that any one of R₁'s at ortho or para positions to the hydroxyl group is a hydrogen atom. n represents an integer of 0 to 4, and when n is 2 or greater, a plurality of R₁'s may be the same or different, and R₁'s positioned ortho with each other, may be combined to form an aliphatic or aromatic 5- to 7-membered ring.

More specifically, R₁ represents a hydrogen atom, a halogen atom (e.g., chlorine, bromine, iodine), an alkyl group [The term "alkyl group" is employed to embrace straight chain, branched chain, or cyclic, and substituted or unsubstituted alkyl groups. Said alkyl group includes an alkyl group (preferably, an alkyl group having 1 to 30 carbon atoms, e.g., methyl, ethyl, n-propyl, isopropyl, t-butyl, n-octyl, eicosyl, 2-ethylhexyl), a cycloalkyl group (preferably, a substituted or unsubstituted cycloalkyl group having 3 to 30 carbon atoms, e.g., cyclohexyl, cyclopentyl, 4-n-dodecylcyclohexyl), and a bicycloalkyl group (preferably, a substituted or unsubstituted bicycloalkyl group having 5 to 30 carbon atoms, namely, a monovalent group of a bicycloalkane having 5 to 30 carbon atoms, from which one hydrogen atom is eliminated, e.g., bicyclo[1,2,2]heptane-2-yl, bicyclo[2,2,2]octane-3-yl). The term "alkyl group" also embraces an alkyl group having a multi-ring structure such as a tricyclic structure. Further, an alkyl moiety of a substituent explained later in this specification (e.g., alkyl moieties of alkylthio groups) also represents an alkyl group within the above shown concept.], an alkenyl group [The term "alkenyl group" is employed to embrace a straight-chain, branched-chain, or cyclic, and substituted or unsubstituted alkenyl group. Said alkenyl group includes an alkenyl group (preferably, a substituted or unsubstituted alkenyl group having 2 to 30 carbon atoms, e.g., vinyl, allyl, prenyl, geranyl, oleyl), a cycloalkenyl group (preferably, a substituted or unsubstituted cycloalkenyl group having 3 to 30 carbon atoms, namely, a monovalent group of a cycloalkene having 3 to 30 carbon atoms, from which one hydrogen atom is eliminated, e.g., 2-cyclopentene-1-yl, 2-cyclohexene-1-yl), and a bicycloalkenyl group (a substituted or unsubstituted bicycloalkenyl group, and preferably, a substituted or unsubstituted bicycloalkenyl group having 5 to 30 carbon atoms, namely, a monovalent group of bicycloalkene having one double bond, from which one hydrogen atom is eliminated, e.g., bicyclo[2,2,1]hepto-2-ene-1-yl, bicyclo[2,2,2]octo-2-ene-4-yl).], an alkynyl group (preferably, a substituted or unsubstituted alkynyl group having 2 to 30 carbon atoms, e.g., ethynyl, propargyl), an aryl group (preferably, a substituted or unsubstituted aryl group having 6 to 30 carbon atoms, e.g., phenyl, p-tolyl, m-chlorophenyl), a heterocyclic group (preferably, a monovalent group of a 5-or 6-membered, substituted or unsubstituted, aromatic or non-aromatic heterocyclic compound, from which one hydrogen atom is eliminated; and more preferably 5- or 6-membered aromatic heterocyclic group having 3 to 30 carbon atoms; e.g., 2-furyl, 2-pyrimidinyl, 2-benzothiazolyl), a cyano group, a hydroxyl group, a nitro group, a carboxyl group, an alkoxy group (preferably, a substituted or unsubstituted alkoxy group having 1 to 30 carbon atoms, e.g., methoxy, ethoxy, isopropoxy, t-butoxy, n-octyloxy), an aryloxy group (preferably, a substituted or unsubstituted aryloxy group having 6 to 30 carbon atoms, e.g., phenoxy, 2-methylphenoxy, 4-t-butylphenoxy), a heterocyclic oxy group (preferably, a substituted or unsubstituted heterocyclic oxy group having 2 to 30 carbon atoms, e.g., 1-phenyltetrazole-5-oxy, 2-tetrahydropyranyloxy), an acyloxy group (preferably, a formyloxy group, a substituted or unsubstituted alkylcarbonyloxy group having 2 to 30 carbon atoms, and a substituted or unsubstituted arylcarbonyloxy group having 6 to 30 carbon atoms, e.g., formyloxy, acetyloxy, pivaloyloxy, stearoyloxy, benzoyloxy, p-methoxyphenylcarbonyloxy), an amino group (preferably, an amino group, a substituted or unsubstituted alkylamino group having 1 to 30 carbon atoms, and a substituted or unsubstituted anilino group having 6 to 30 carbon atoms, e.g., amino, methylamino, dimethylamino, anilino, N-methyl-anilino, diphenylamino), an acylamino group (preferably, a formylamino group, a substituted or unsubstituted alkylcarbonylamino group having 1 to 30 carbon atoms, and a substituted or unsubstituted arylcarbonylamino group having 6 to 30 carbon atoms, e.g., formylamino, acetylamino, pivaloylamino, lauroylamino, benzoylamino), an aminocarbonylamino group (preferably, a substituted or unsubstituted aminocarbonylamino group having 1 to 30 carbon atoms, e.g., carbamoylamino, N,N-dimethylaminocarbonylamino, N,N-diethylaminocarbonylamino, morpholinocarbonylamino), an alkoxycarbonylamino group (preferably, a substituted or unsubstituted alkoxycarbonylamino group having 2 to 30 carbon atoms, e.g., methoxycarbonylamino, ethoxycarbonylamino, t-butoxycarbonylamino, n-octadecyloxycarbonylamino), a sulfamoylamino group (preferably, a substituted or unsubstituted sulfamoylamino group having 0 to 30 carbon atoms, e.g., sulfamoylamino, N,N-dimethylaminosulfonylamino, N-n-octylaminosulfonylamino), an alkyl- or aryl-sulfonylamino group (preferably, a substituted or unsubstituted alkylsulfonylamino group having 1 to 30 carbon atoms, and a substituted or unsubstituted arylsulfonylamino group having 6 to 30 carbon atoms, e.g., methylsulfonylamino, butylsulfonylamino, phenylsulfonylamino, p-methylphenylsulfonylamino), a mercapto group, an alkylthio group (preferably, a substituted or unsubstituted alkylthio group having 1 to 30 carbon atoms, e.g., methylthio, ethylthio, n-hexadecylthio), an arylthio group (preferably, a substituted or unsubstituted arylthio group having 6 to 30 carbon atoms, e.g., phenylthio, p-chlorophenylthio, m-methoxyphenylthio), a heterocyclic thio group (preferably, a substituted or unsubstituted heterocyclic thio group having 2 to 30 carbon atoms, e.g., 2-benzothiazolylthio, 1-phenyltetrazole-5-yl thio), a sulfamoyl group (preferably, a substituted or unsubstituted sulfamoyl group having 0 to 30 carbon atoms, e.g., N-ethylsulfamoyl, N,N-dimethylsulfamoyl), a sulfo group, an alkyl- or aryl-sulfinyl group (preferably, a substituted or unsubstituted alkylsulfinyl group having 1 to 30 carbon atoms, and a substituted or unsubstituted arylsulfinyl group having 6 to 30 carbon atoms, e.g., methylsulfinyl, ethylsulfinyl, phenylsulfinyl, p-methylphenylsulfinyl), an alkyl- or aryl-sulfonyl group (preferably, a substituted or unsubstituted alkylsulfonyl group having 1 to 30 carbon atoms, and a substituted or unsubstituted arylsulfonyl group having 6 to 30 carbon atoms, e.g., methylsulfonyl, ethylsulfonyl, phenylsulfonyl, p-methylphenylsulfonyl), an acyl group (preferably, a formyl group, a substituted or unsubstituted alkylcarbonyl group having 2 to 30 carbon atoms, and a substituted or unsubstituted arylcarbonyl group having 7 to 30 carbon atoms, e.g., acetyl, pivaloyl, 2-chloroacetyl, strearoyl, benzoyl), an aryloxycarbonyl group (preferably, a substituted or unsubstituted aryloxycarbonyl group having 7 to 30 carbon atoms, e.g., phenoxycarbonyl, p-t-butylphenoxycarbonyl), an alkoxycarbonyl group (preferably, a substituted or unsubstituted alkoxycarbonyl group having 2 to 30 carbon atoms, e.g., methoxycarbonyl, ethoxycarbonyl, t-butoxycarbonyl, n-octadecyloxycarbonyl), a carbamoyl group (preferably, a substituted or unsubstituted carbamoyl group having 1 to 30 carbon atoms, e.g., carbamoyl, N-methylcarbamoyl, N,N-dimethylcarbamoyl, N,N-di-n-octylcarbamoyl), an imido group (preferably, N-succinimido, N-phthalimido), a phosphino group (preferably, a substituted or unsubstituted phosphino group having 2 to 30 carbon atoms, e.g., dimethylphosphino, diphenylphosphino, methylphenoxyphosphino), a phosphinyl group (preferably, a substituted or unsubstituted phosphinyl group having 2 to 30 carbon atoms, e.g., phosphinyl, dioctyloxyphosphinyl, diethoxyphosphinyl), a phosphinylamino group (preferably, a substituted or unsubstituted phosphinyl amino group having 2 to 30 carbon atoms, e.g., dimethoxyphosphinylamino, dimethylaminophosphinylamino), or a silyl group (preferably, a substituted or unsubstituted silyl group having 3 to 30 carbon atoms, e.g., trimethylsilyl, t-butyldimethylsilyl, phenyldimethylsilyl).

In formula, R₂ represents a hydrogen atom, an alkyl group (the term "alkyl group" referred to in the present invention means a straight chain, branched chain, or cyclic, and substituted or unsubstituted alkyl group), an acyl group, an alkylsulfonyl group, an arylsulfonyl group, an alkoxycarbonyl group, an alkylcarbamoyl group, or an arylcarbamoyl group.

The alkyl group represented by R₂ has preferably 1 to 32, more preferably 1 to 22 carbon atoms. Specific examples of the alkyl group include methyl, ethyl, propyl, isopropyl, 2-propenyl, 2-propinyl, butyl, isobutyl, hexyl, cyclohexyl, 2-ethylhexyl, octyl, decyl, dodecyl, hexadecyl, and octadecyl groups.

The cycloalkyl group represented by R₂ has preferably 3 to 12, more preferably 3 to 6 carbon atoms. Specific examples of the cycloalkyl group include cyclopropyl and cyclohexyl groups.

The acyl group represented by R₂ has preferably 1 to 32, more preferably 1 to 22 carbon atoms. Specific examples of the acyl group include formyl, acetyl, propanoyl, butanoyl, isobutanoyl, hexanoyl, octanoyl, decanoyl, dodecanoyl, hexadecanoyl, octadecanoyl, and benzoyl groups.

The alkylsulfonyl group represented by R₂ has preferably 1 to 18, more preferably 1 to 4 carbon atoms. Specific examples of the alkylsulfonyl group include methylsulfonyl, ethylsulfonyl, propylsulfonyl, and butylsulfonyl groups.

The arylsulfonyl group represented by R₂ has preferably 6 to 24, more preferably 6 to 18 carbon atoms. Specific examples of the arylsulfonyl group include phenylsulfonyl, and p-toluenesulfonyl groups.

The alkoxycarbonyl group represented by R₂ has preferably 1 to 32, more preferably 1 to 22 carbon atoms. Specific examples of the alkoxycarbonyl group include methoxycarbonyl, ethoxycarbonyl, isobutyloxycarbonyl, and octyloxycarbonyl groups.

The alkylcarbamoyl group represented by R₂ has preferably 1 to 32, more preferably 1 to 22 carbon atoms. Specific examples of the alkylcarbamoyl group include methylcarbamoyl, ethylcarbamoyl, and octylcarbamoyl groups.

The arylcarbamoyl group represented by R₂ has preferably 7 to 24, more preferably 7 to 16 carbon atoms. Specific examples of the arylcarbamoyl group include phenylcarbamoyl, p-tolylcarbamoyl groups.

Among the foregoing substituents, substituents that can be further substituted, may be substituted by the following substituent(s). Those further substituents can be mentioned are:
a halogen atom (e.g., fluorine, chlorine, bromine, iodine), a hydroxyl group, a carboxyl group, a sulfo group, a cyano group, a nitro group, an alkyl group (e.g., methyl, ethyl, hexyl), an alkenyl group (e.g., vinyl), an alkynyl group (e.g., ethynyl), a fluoroalkyl group (e.g., trifluoromethyl), an aryl group (e.g., phenyl, tolyl, naphthyl), an alkoxy group (e.g., methoxy, ethoxy, octyloxy), an aryloxy group (e.g., phenoxy, naphthyloxy), an alkylthio group (e.g., methylthio, butylthio), an arylthio group (e.g., phenylthio), an amino group (e.g., amino, N-methylamino, N,N-dimethylamino, N-phenylamino), an acyl group (e.g., acetyl, propionyl, benzoyl), an alkyl or arylsulfonyl group (e.g., methylsulfonyl, phenylsulfonyl), an acylamino group (e.g., acetylamino, benzoylamino), an alkyl- or aryl-sulfonylamino group (e.g., methanesulfonylamino, benzenesulfonylamino), a carbamoyl group (e.g., carbamoyl, N-methylaminocarbonyl, N,N-dimethylamino carbonyl, N-phenylaminocarbonyl), a sulfamoyl group (e.g., sulfamoyl, N-methylaminosulfonyl, N,N-dimethylaminosulfonyl, N-phenylaminosulfonyl), an alkoxycarbonyl group (e.g., methoxycarbonyl, ethoxycarbonyl, octyloxycarbonyl), an aryloxycarbonyl group (e.g., phenoxycarbonyl, naphthyloxycarbonyl), an acyloxy group (e.g., acetyloxy, benzoyloxy), an alkoxycarbonylamino group (e.g., methoxycarbonylamino, butoxycarbonylamino), an aminocarbonylamino group (e.g., N-methylaminocarbonylamino, N-phenylaminocarbonylamino).

Specific examples of the compounds represented by formulas (II) and (IV), which are suitable for production according to the production method of the present invention, are shown below. However, the present invention is not limited thereto.

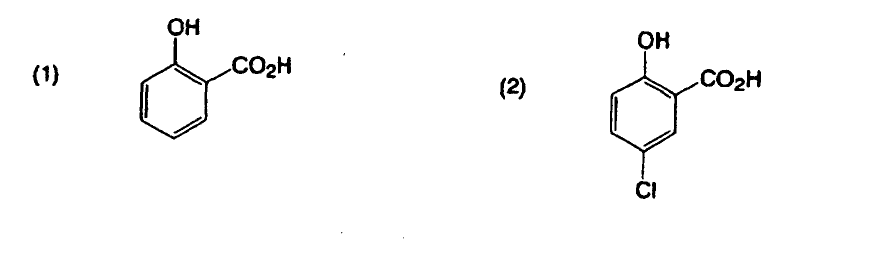

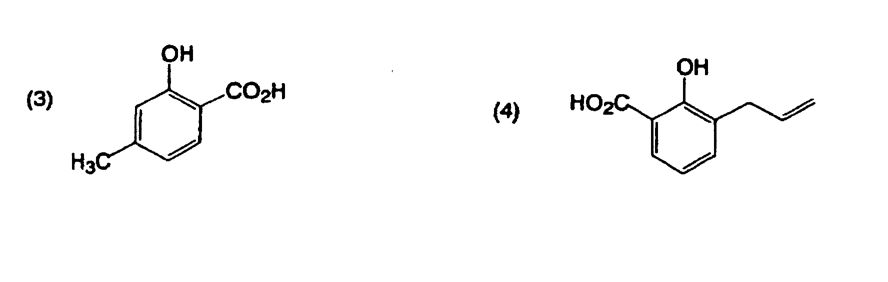

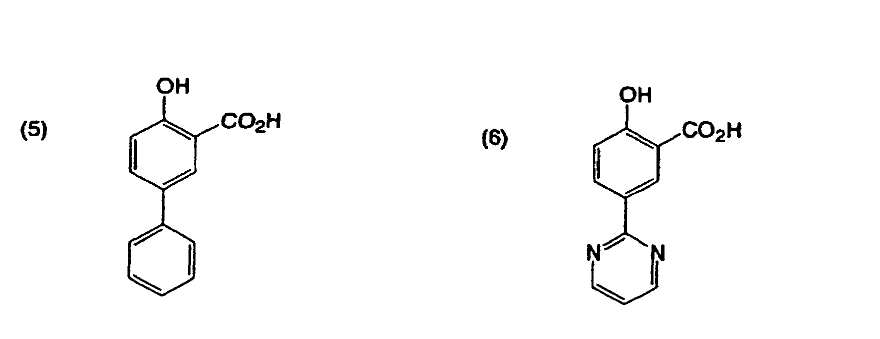

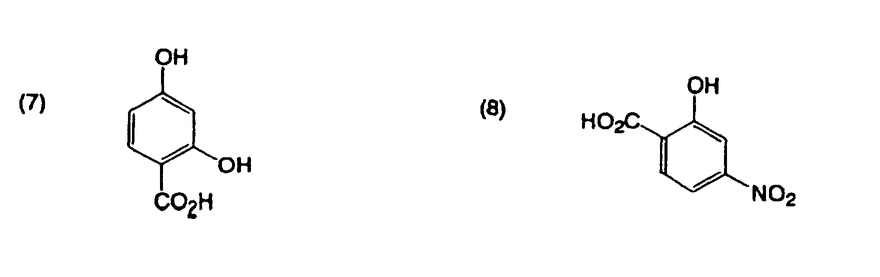

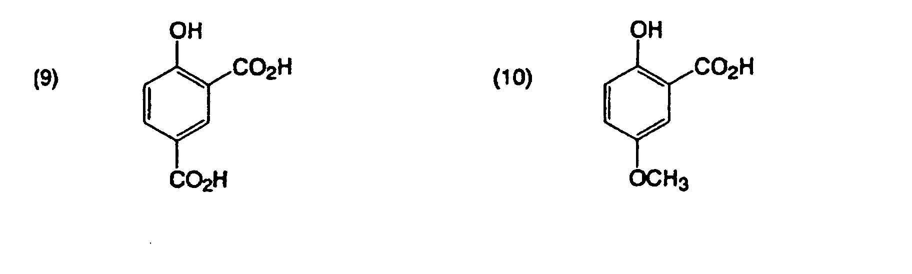

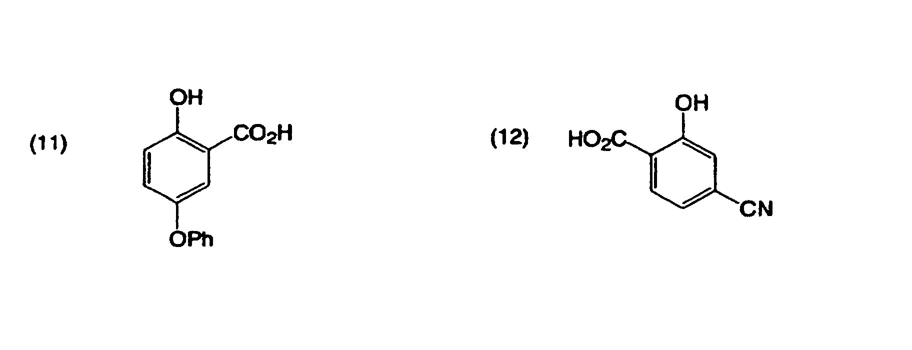

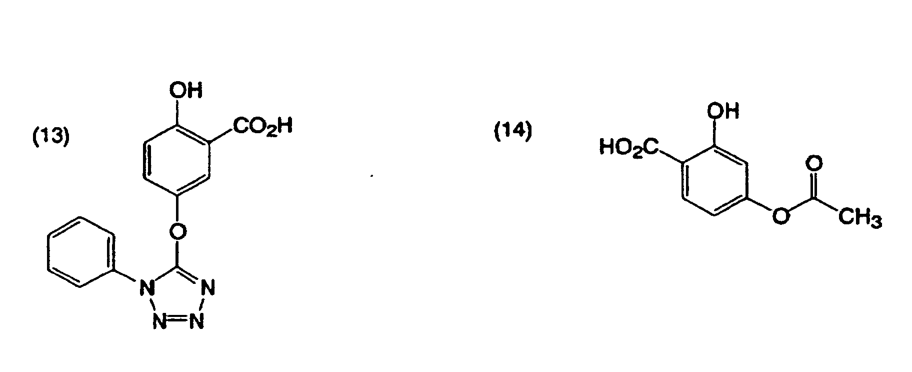

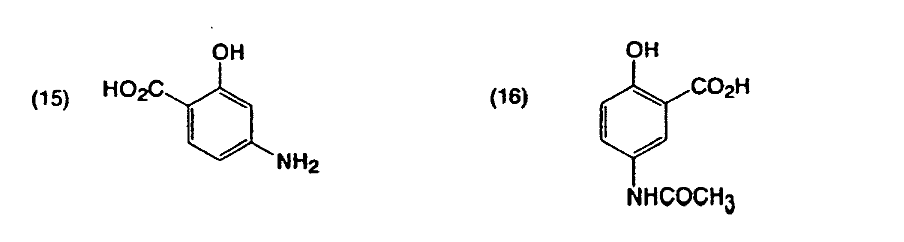

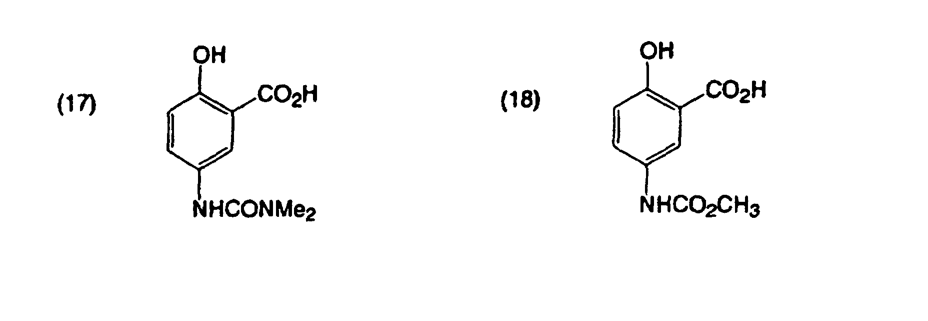

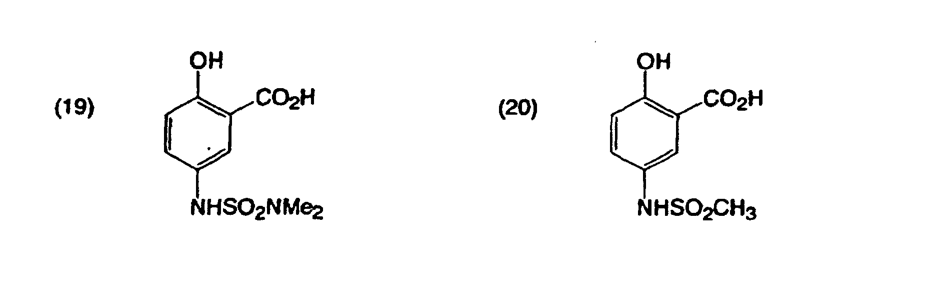

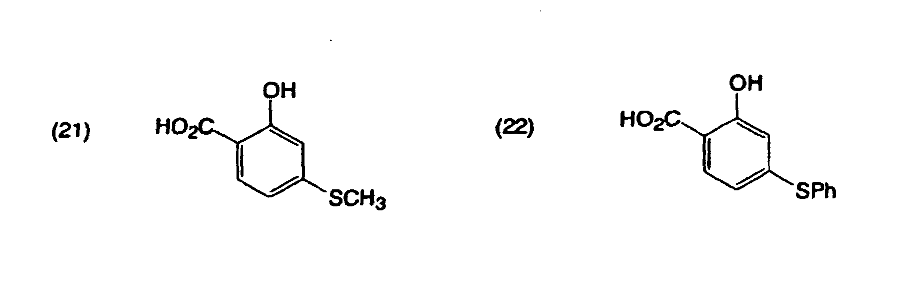

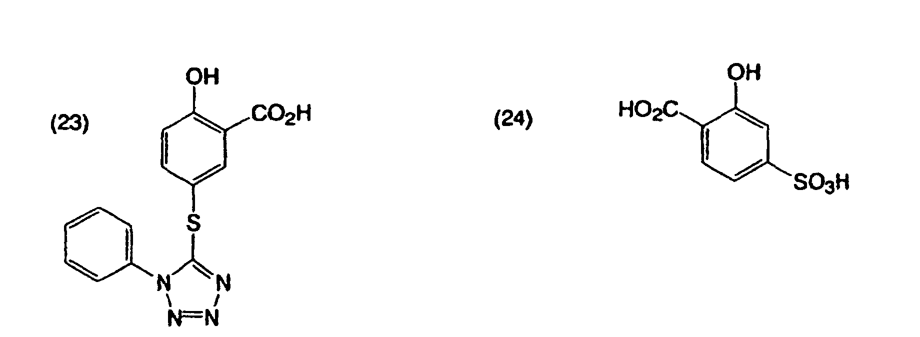

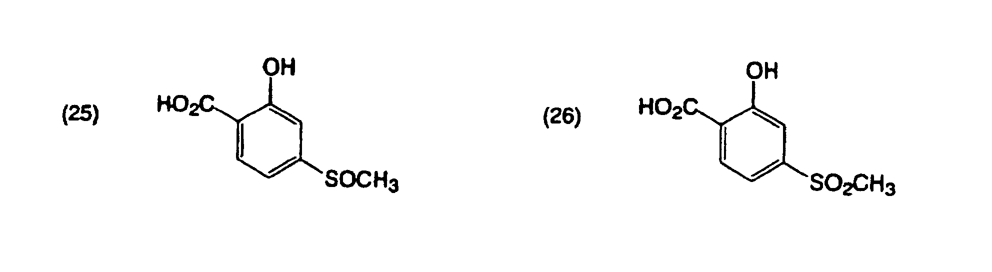

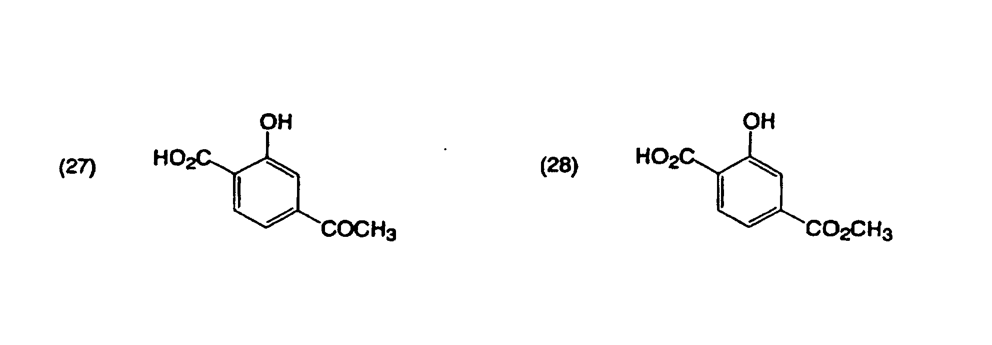

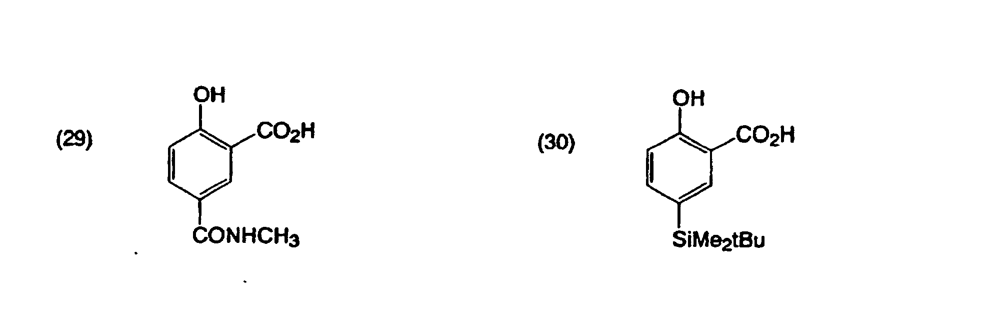

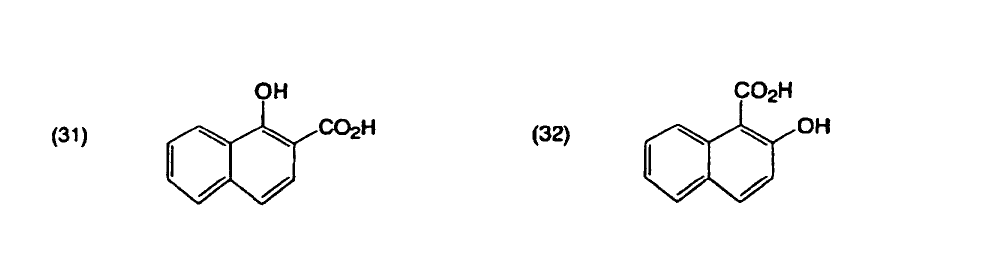

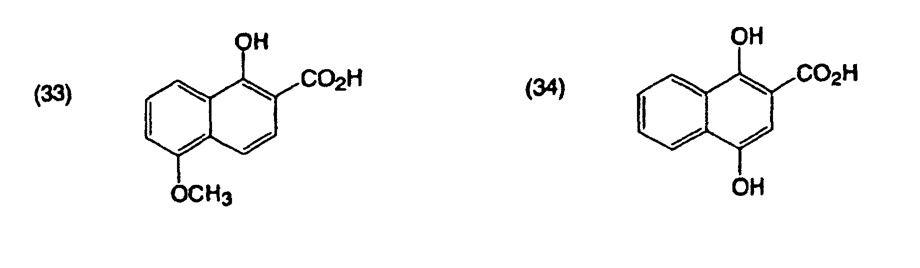

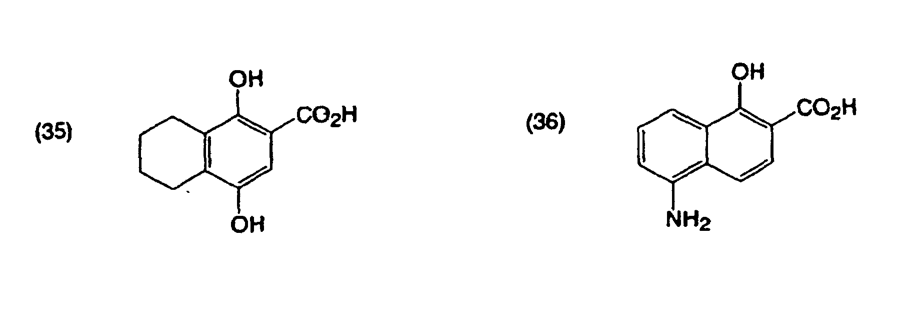

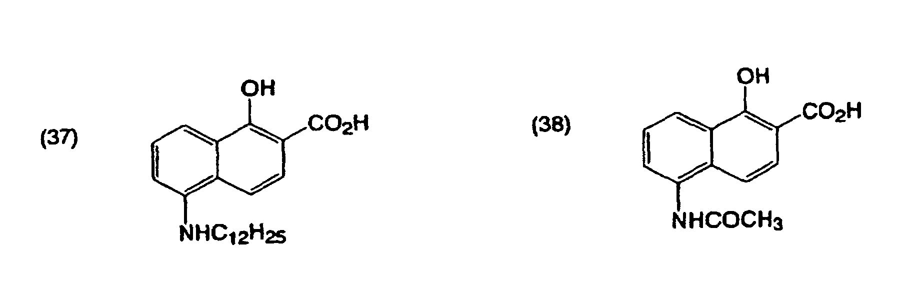

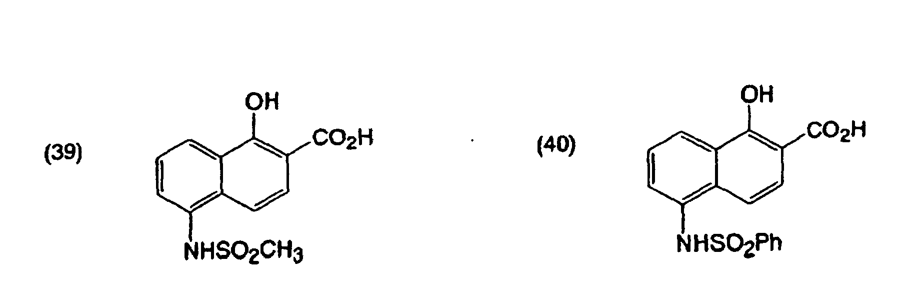

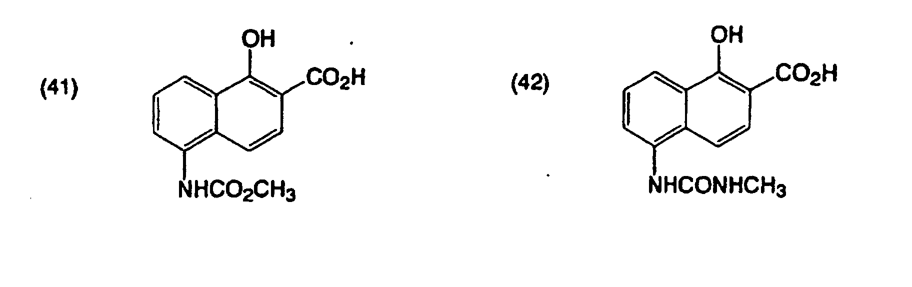

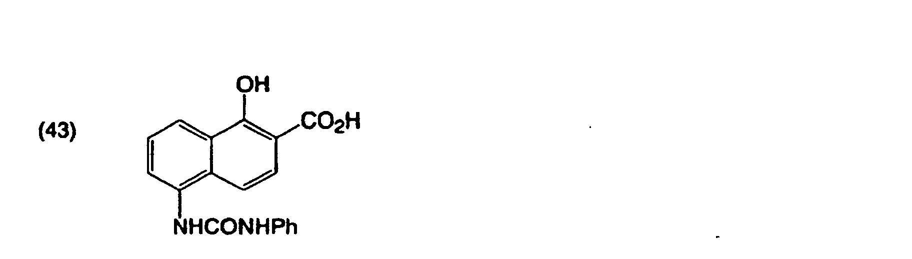

Next, a method for producing the compound represented by formula (II) is described in detail.

The compound represented by formula (II) is synthesized by reacting a compound represented by formula (I) and carbon dioxide, in the presence of sodium t-butoxide, in an aprotic polar solvent.

The amount of the aprotic polar solvent to be used is not limited in particular, and the solvent may be present in an amount sufficient to dissolve raw materials for the reaction at a minimum. However, preferably, the amount of the solvent is 1 or more times, and more preferably 2 to 10 times that of the compound of formula (I), in terms of weight ratio.

The molar ratio of sodium t-butoxide to the compound represented by formula (I) is generally in the range of 1 to 10, preferably in the range of 1 to 6. The sodium t-butoxide can be used as a mixed base, to which potassium carbonate is added.

The molar ratio of potassium carbonate to sodium t-butoxide is generally in the range of 0.1 to 10, and preferably in the range of 0.1 to 2.

As an aprotic solvent for use in the reaction, N,N-dimethylformamide, N,N-dimethylacetamide, N-methylpyrrolidone, N,N'-dimethylimidazolidone, or the like can be mentioned, and they may be used singly or as a mixed solvent. Among these solvents, N,N-dimethylacetamide, and N-methylpyrrolidone are preferred.

The reaction temperature at which a compound represented by formula (I) reacts with a carbon dioxide, is preferably in the range of 20 °C to 200 °C, and more preferably in the range of 20 °C to 150 °C.

The pressure of carbon dioxide in the reaction of the compound represented by formula (I) and said carbon dioxide is preferably in the range of 0.5 to 50 atm, and more preferably in the range of 5 to 20 atm.

The reaction time required for the reaction of the compound represented by formula (I) and carbon dioxide, is preferably in the range of 1 to 12 hours, and more preferably in the range of 1 to 6 hours.

As a reaction procedure, the procedures can be taken are:
a procedure wherein a compound represented by formula (I) is heated and stirred, in the presence of sodium t-butoxide, in an aprotic polar solvent, under a nitrogen atmosphere, and after elevating a temperature in the reaction system, said compound is allowed to react with carbon dioxide; a procedure wherein a compound represented by formula (I) is heated and stirred, in the presence of sodium t-butoxide, in an aprotic polar solvent, under a carbon dioxide atmosphere, and after elevating a temperature, said carbon dioxide may be pressurized; or alternatively, a procedure wherein a compound represented by formula (I) is heated to elevate a temperature, in pressurized carbon dioxide, in the presence of sodium t-butoxide, in an aprotic polar solvent.

Among the foregoing methods, it is preferable to use a method wherein a compound represented by formula (I) is heated and stirred under a nitrogen atmosphere, in an aprotic polar solvent, in the presence of t-butoxide, and then allowed to react with carbon dioxide after elevating a temperature of the reaction system.

The production of the compound represented by formula (IV) from the compound represented by formula (III) can be conducted with the same reaction conditions as described above.

The production method according to the present invention exerts an excellent effect that an aromatic carboxylic acid having a carboxyl group introduced at the 2- or 4-position can be obtained, in a high yield under a relatively moderate condition, and by a simple operation.

Next, the present invention is described in more detail on the basis of the following examples, but the invention is not limited to those.

### EXAMPLE

### Example 1

### Synthesis of Compound (37)

In a reaction vessel having a capacity of 200 ml, were placed 5 g of 5-dodecylamino-1-naphthol, 4.4 g of sodium t-butoxide, and 25 ml of N-methylpyrrolidone, under a nitrogen atmosphere. The resultant mixture was heated to elevate a temperature to 120 °C, and stirred for 3 hours under a carbon dioxide atmosphere of 9 atm, and then cooled to 40 °C. The resultant liquid was poured into a mixture of ethyl acetate (50 ml)/water (50 ml), and then concentrated hydrochloric acid was added until the pH of the aqueous layer reached 8.5. To the thus-obtained mixed solution, 0.5 g of active carbon was added, and stirred for 15 minutes, followed by filtration using celite.
After separation, an organic layer was concentrated so as to become about 20 ml, and then poured into a mixture of water (30 ml)/concentrated hydrochloric acid (1 ml). To the thus-obtained mixture, 15 ml of hexane was added, cooled to 15 °C while slowly stirring, and then allowed to stand for 30 minutes at the temperature. Crystals were collected by filtration, and washed by dashing ethyl acetate/hexane (=1/1) and acetonitrile thereon. Thereafter, they were dried to obtain 5.5 g compound (37).
(Identification of the compound was performed by elemental analysis, NMR, and Mass spectrum.)
¹H NMR (300MHz, DMSO-d₆)
δ: 0.84 (t, J = 6.6 Hz, 3H), 1.22 (bs, 18H), 1.55-1.70 (m, 2H), 3.16 (t, J = 6.9 Hz, 2H), 3.35 (bs, 1H), 6.10 (bs, 1H), 6.63 (d, J = 7.5 Hz, 1H), 7.32 (dd, J = 7.5, 8.1 Hz, 1H), 7.41 (d, J = 8.1 Hz, 1H), 7.59 (d, J = 9.1 Hz, 1H), 7.63 (d, J = 9.1 Hz, 1H), 12.50 (bs, 1H)

| Elemental Analysis | | | | |
|---|---|---|---|---|
| Calculated | C 74.36% | H 8.95% | N 3.77% | O 12.92% |
| Obtained | C 74.18% | H 8.96% | N 3.76% | O 13.10% |

Next, as comparative examples, synthesis's in which sodium methoxide is used as a base in place of sodium t-butoxide are described.

### Comparative example 1

### Synthesis of Compound (37)

In a reaction vessel having a capacity of 200 ml were placed 5 g of 5-dodecylamino-1-naphthol, 2.5 g of sodium methoxide, and 25 ml of N-methylpyrrolidone, under a nitrogen atmosphere. The resultant mixture was heated to elevate a temperature to 120 °C, and stirred for 3 hours under a carbon dioxide atmosphere of 9 atm.

### Comparative example 2

### Synthesis of Compound (37) (Method described in JP-A-5-194314)

The mixture as in comparative example 1 was prepared, under the same conditions as in comparative example 1, then the resultant mixture was heated to elevate a temperature to 180 °C, and stirred for 1 hour. After having eliminated the formed methanol, the mixture and carbon dioxide were reacted.

The reaction rate in comparative examples 1 and 2 are shown below. The "reaction rate" is a value obtained by converting absorbance obtained by measurement of HPLC into a molar ratio.

**Table 1**

| | Base | Reaction rate |
|---|---|---|
| Comparative example 1 | CH₃ONa | 68% |
| Comparative example 2 | CH₃ONa | 93% |
| Example 1 | t-BuONa | 98% |

As shown in the foregoing comparative examples, the use of sodium methoxide requires a procedure to eliminate the formed methanol. Contrary, the use of sodium t-butoxide as defined in the present invention does not require to eliminate any alcohol. Therefore, it is considered that the method of the present invention is advantageous for industrial purposes in the foregoing point.

Further, the results that were obtained by other comparative examples 3 and 4, in which a reaction was conducted in the same manner as in Example 1, except that a solvent and/or a base were changed, are shown below.

**Table 2**

| | Solvent | Base | Equivalent weight | Yield |
|---|---|---|---|---|
| Comparative example 3 | Kerosine | K₂CO₃ | 8 | 6% |
| Comparative example 4 | NMP | K₂CO₃ | 8 | 63% |
| Example 1 | NMP | t-BuONa | 3 | 98% |

| | | | | |
|---|---|---|---|---|
| ∗ NMP = N-methylpyrrolidone | | | | |

### Example 2

### Synthesis of Compound (36)

In a reaction vessel having a capacity of 500 ml, were placed 10 g of 5-amino-1-naphthol, 18.1 g of sodium t-butoxide, 26.1 g of potassium carbonate, and 50 ml of N-methylpyrrolidone, under a nitrogen atmosphere. The resultant mixture was heated to elevate a temperature to 120 °C, and stirred for 3 hours under a carbon dioxide atmosphere of 9 atm. After cooling to 50 °C, the thus-obtained liquid was poured into a mixture of ethyl acetate (100 ml)/water (100 ml). Further, 1 g of active carbon was added and stirred for 15 minutes, followed by filtration using celite. To the resultant mixed solution, concentrated hydrochloric acid was added until the pH of the aqueous layer reached 3. Thereafter, the mixture was cooled to 15 °C while slowly stirring, and then allowed to stand for 30 minutes at the temperature. Crystals were collected by filtration, and washed by dashing a mixture of ethyl acetate/hexane (=1/1) and acetonitrile thereon. Thereafter, they were dried to obtain a hydrochloride of compound (36) (13.9 g).

The yield of the compound (36) described in European Patent No. 927153 was 78%. Therefor, it is understood that, by the method in accordance with the present invention (yield: 92%), the same compound was manufactured with a simple method in higher yield in comparison with the method of the European patent.

### Example 3

### Synthesis of Compound (31)

In a reaction vessel having a capacity of 500 ml, were placed 5 g of 1-naphthol, 6.7 g of sodium t-butoxide, and 50 ml of N-methylpyrrolidone under a nitrogen atmosphere. The resultant mixture was heated to elevate a temperature to 50 °C. After adding carbon dioxide, the mixture was stirred at 85 °C for 3 hours under a carbon dioxide atmosphere of 10 atm. After cooling to 50 °C, the thus-obtained liquid was poured into a mixture of ethyl acetate (50 ml)/water (50 ml). Concentrated hydrochloric acid was added to the mixed solution until the pH of the aqueous layer reached 3. After separation, thus-obtained oily layer was concentrated. To the resultant concentrate, concentrated hydrochloric acid was added while slowly stirring, cooled to 15 °C, and then allowed to stand for 30 minutes at the temperature. Crystals were collected by filtration, and washed by dashing water thereon. Thereafter, they were dried to obtain 6.4 g of compound (31).

The yields in reactions for manufacturing compound (31) from 1-naphthol, which were described in literatures, are shown below as comparative examples 5 to 8. In these comparative examples 5 to 8, a Na or K salt of 1-naphthol was initially prepared by reacting 1-naphthol and the corresponding base, then fully dried, and thereafter these salts were utilized in the reaction.

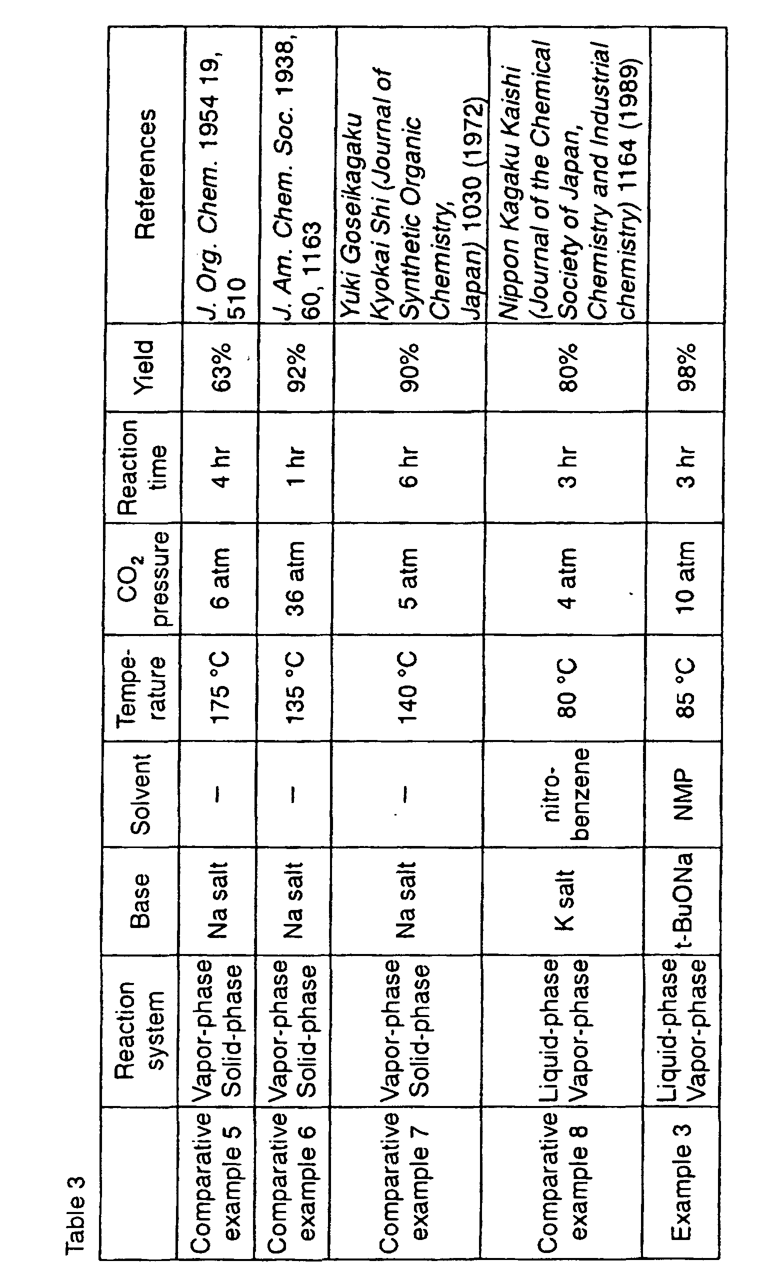

### Example 4

### Synthesis of Compound (3)

In a reaction vessel having a capacity of 500 ml, were placed 5 g of 3-methylphenol, 8.9 g of sodium t-butoxide, and 50 ml of N-methylpyrrolidone, under a nitrogen atmosphere. The resultant mixture was heated to elevate a temperature to 50 °C. After adding carbon dioxide, the mixture was stirred at 120 °C for 3 hours under a carbon dioxide atmosphere of 10 atm. After cooling to 50 °C, the thus-obtained liquid was poured into a mixed solvent of ethyl acetate (50 ml)/water (50 ml). Concentrated hydrochloric acid was added to the mixture until the pH of the aqueous layer reached 3. After separation, thus-obtained oily layer was concentrated. To the resultant concentrate, concentrated hydrochloric acid was added while slowly stirring, cooled to 15 °C, and then allowed to stand for 30 minutes at the temperature. Crystals were collected by filtration, and washed by dashing water thereon. Thereafter, they were dried to obtain 6.5 g of compound (3).

From the results obtained by the foregoing examples and comparative examples, it is understood that according to the production method of the present invention, a carboxyl group can be introduced at the 2- or 4-position of phenols or naphthols, under a relatively moderate condition and in the manner advantageous to industrial production.

Having described our invention as related to the present embodiments, it is our intention that the invention not be limited by any of the details of the description, unless otherwise specified, but rather be construed broadly within its spirit and scope as set out in the accompanying claims.

## Claims

1. A method for producing a compound represented by formula (II), comprising reacting a compound represented by formula (I) and carbon dioxide, in the presence of sodium t-butoxide, in an aprotic polar solvent, thereby introducing a carboxyl group at the 2- or 4-position thereof: 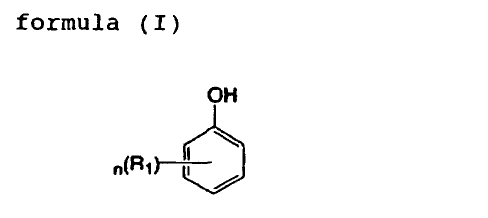 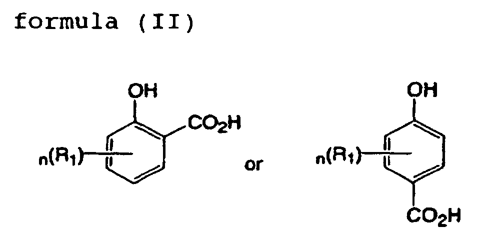 wherein R₁ represents a substituent, with a proviso that at least one of the R₁'s at ortho or para position to the hydroxyl group is a hydrogen atom; n represents an integer of 0 to 4, and when n is 2 or greater, a plurality of R₁'s may be the same or different, and R₁'s positioned ortho with each other, may be combined to form an aliphatic or aromatic 5-to 7-membered ring.

2. The method as claimed in claim 1, wherein the aprotic solvent is selected from a group consisting of N,N-dimethylformamide, N,N-dimethylacetamide, N-methylpyrrolidone, and N,N'-dimethylimidazolidone.

3. The method as claimed in claim 1, wherein the aprotic polar solvent is used in an amount of 2 to 10 times the amount of the compound represented by formula (I).

4. The method as claimed in claim 1, wherein a molar ratio of sodium t-butoxide to the compound represented by formula (I) is in the range of 1 to 10.

5. The method as claimed in claim 1, comprising steps of heating and stirring the compound represented by formula (I), under a nitrogen atmosphere, in the aprotic solvent, in the presence of sodium t-butoxide; elevating a temperature; and then reacting the compound with carbon dioxide.

6. A method for producing a compound represented by formula (IV), comprising reacting a compound represented by formula (III) and carbon dioxide, in the presence of sodium t-butoxide, in an aprotic polar solvent, thereby introducing a carboxyl group at the 2-position thereof: 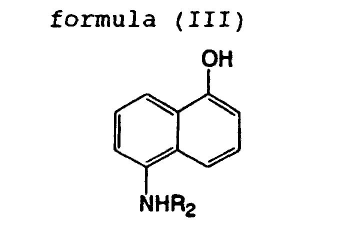 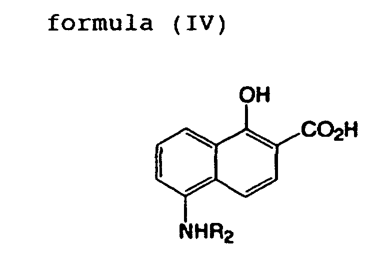 wherein R₂ represents a hydrogen atom, an alkyl group, an acyl group, an alkylsulfonyl group, an arylsulfonyl group, an alkoxycarbonyl group, an alkylcarbamoyl group, or an arylcarbamoyl group.

7. The method as claimed in claim 6, wherein the aprotic solvent is selected from a group consisting of N,N-dimethylformamide, N,N-dimethylacetamide, N-methylpyrrolidone, and N,N'-dimethylimidazolidone.

8. The method as claimed in claim 6, wherein the aprotic polar solvent is used in an amount of 2 to 10 times the amount of the compound represented by formula (III).

9. The method as claimed in claim 6, wherein a molar ratio of sodium t-butoxide to the compound represented by formula (III) is in the range of 1 to 10.

10. The method as claimed in claim 6, comprising steps of heating and stirring the compound represented by formula (III), under a nitrogen atmosphere, in the aprotic solvent, in the presence of sodium t-butoxide; elevating a temperature; and then reacting the compound with carbon dioxide.

## Patentansprüche

1. Verfahren zur Herstellung einer Verbindung der Formel (II), das die Umsetzung einer Verbindung der Formel (I) mit Kohlendioxid in Gegenwart von Natrium-t-butoxid in einem aprotischen polaren Lösungsmittel umfasst, wodurch eine Carboxylgruppe in der 2- oder 4-Position eingeführt wird: 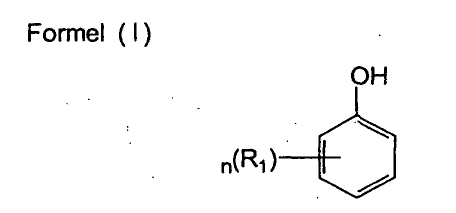 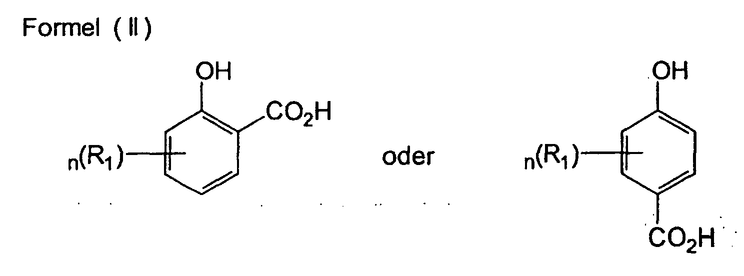 worin R₁ einen Substituenten darstellt, mit der Massgabe, dass mindestens ein R₁ in der ortho- oder para-Position zur Hydroxylgruppe ein Wasserstoffatom ist; n ist eine ganze Zahl von 0-4, und wenn n 2 oder grösser ist, können die mehreren R₁ identisch oder voneinander verschieden sein, und die zueinander orthoständigen R₁-Gruppen können unter Bildung eines aliphatischen oder aromatischen 5- bis 7-gliedrigen Rings miteinander verbunden sein.

2. Verfahren gemäss Anspruch 1, worin das aprotische Lösungsmittel ausgewählt ist aus N,N-Dimethylformamid, N,N-Dimethylacetamid, N-Methylpyrrolidon und N,N'-Dimethylimidazolidon.

3. Verfahren gemäss Anspruch 1, worin das aprotische polare Lösungsmittel in einer Menge verwendet wird, die dem 2- bis 10-fachen der Menge der Verbindung der Formel (I) entspricht.

4. Verfahren gemäss Anspruch 1, worin das Molverhältnis von Natrium-t-butoxid zur Verbindung der Formel (I) im Bereich von 1 bis 10 liegt.

5. Verfahren gemäss Anspruch 1, das die Schritte des Erwärmens und Rührens der Verbindung der Formel (I) unter einer Stickstoffatmosphäre in dem aprotischen Lösungsmittel in Gegenwart von Natrium-t-butoxid, Anheben der Temperatur und anschliessende Umsetzung der Verbindung mit Kohlendioxid umfasst.

6. Verfahren zur Herstellung einer Verbindung der Formel (IV), das die Umsetzung einer Verbindung der Formel (III) mit Kohlendioxid in Gegenwart von Natrium-t-butoxid in einem aprotischen Lösungsmittel umfasst, wodurch eine Carboxylgruppe in die 2-Position eingeführt wird: 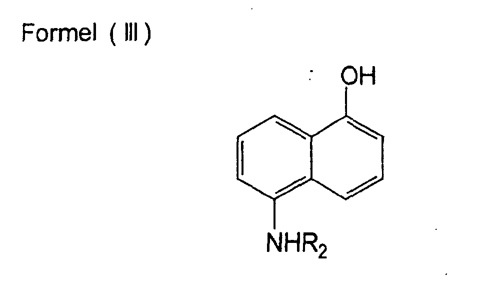 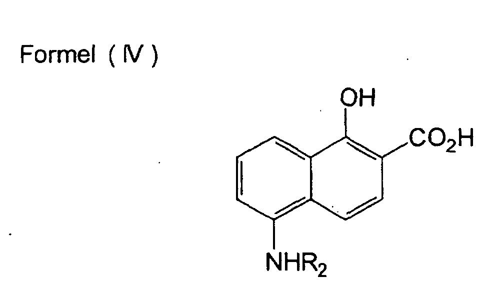 worin R₂ ein Wasserstoffatom, eine Alkylgruppe, eine Acylgruppe, eine Alkylsulfonylgruppe, eine Arylsulfonylgruppe, eine Alkoxycarbonylgruppe, eine Alkylcarbamoylgruppe oder eine Arylcarbamoylgruppe ist.

7. Verfahren gemäss Anspruch 6, worin das aprotische Lösungsmittel ausgewählt ist aus N,N-Dimethylformamid, N,N-Dimethylacetamid, N-Methylpyrrolidon und N,N'-Dimethylimidazolidon.

8. Verfahren gemäss Anspruch 6, worin das aprotische polare Lösungsmittel in einer Menge verwendet wird, die dem 2- bis 10-fachen der Menge der Verbindung der Formel (III) entspricht.

9. Verfahren gemäss Anspruch 6, worin das Molverhältnis von Natrium-t-butoxid zur Verbindung der Formel (III) im Bereich von 1 bis 10 liegt.

10. Verfahren gemäss Anspruch 6, das die Schritte des Erwärmens und Rührens der Verbindung der Formel (III) unter einer Stickstoffatmosphäre in dem aprotischen Lösungsmittel in Gegenwart von Natrium-t-butoxid, Anheben der Temperatur und anschliessende Umsetzung der Verbindung mit Kohlendioxid umfasst.

## Revendications

1. Procédé pour produire un composé représenté par la formule (II), comprenant de faire réagir un composé représenté par la formule (I) et du dioxyde de carbone, en présence de t-butoxyde de sodium, dans un solvant polaire aprotique, introduisant de ce fait un groupe carboxyle à la position 2 ou 4 de celui-ci : 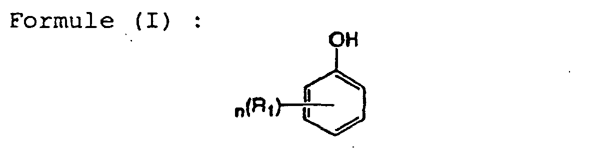 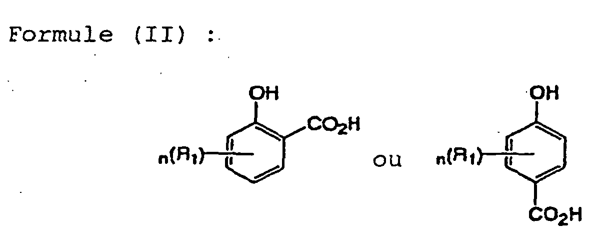 dans lesquelles R₁ représente un substituant, avec la condition que au moins un des R₁ à la position ortho ou para par rapport au groupe hydroxyle soit un atome d'hydrogène ; n représente un nombre entier de 0 à 4, et lorsque n est 2 ou plus grand, une pluralité de R₁ peuvent être les mêmes ou différents, et les R₁ positionnés en ortho les uns aux autres peuvent être combinés afin de former un cycle aromatique ou aliphatique a de 5 à 7 membres.

2. Procédé comme revendiqué dans la revendication 1, dans lequel le solvant aprotique est choisi à partir d'un groupe constitué par du N,N-diméthylformamide, du N,N-diméthylacétamide, de la N-méthylpyrrolidone, et de la N,N'-diméthylimidazolidone.

3. Procédé comme revendiqué dans la revendication 1, dans lequel le solvant polaire aprotique est utilisé en une quantité de 2 à 10 fois la quantité du composé représenté par la formule (I).

4. Procédé comme revendiqué dans la revendication 1, dans lequel un rapport molaire de t-butoxyde de sodium sur le composé représenté par la formule (I) se situe dans la gamme de 1 à 10.

5. Procédé comme revendiqué dans la revendication 1, comprenant des étapes de chauffer et d'agiter le composé représenté par la formule (I), sous une atmosphère d'azote, dans le solvant aprotique, en présence de t-butoxyde de sodium ; d'élever une température ; et ensuite de faire réagir le composé avec du dioxyde de carbone.

6. Procédé pour produire un composé représenté par la formule (IV), comprenant de faire réagir un composé représenté par la formule (III) et du dioxyde de carbone, en présence de t-butoxyde de sodium, dans un solvant polaire aprotique, introduisant de ce fait un groupe carboxyle à la position 2 de celui-ci : 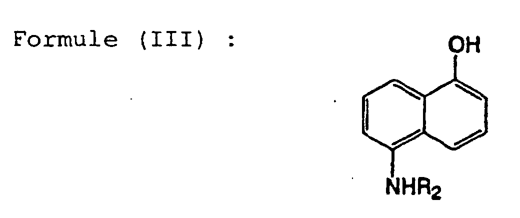 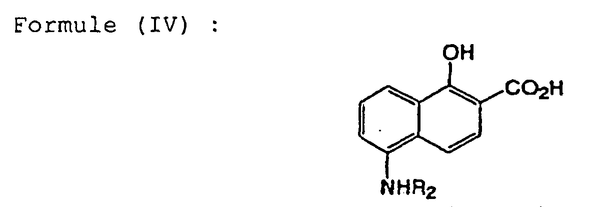 dans lesquelles R₂ représente un atome d'hydrogène, un groupe alkyle, un groupe acyle, un groupe alkylsulfonyle, un groupe arylsulfonyle, un groupe alcoxysulfonyle, un groupe alkylcarbamoyle, ou un groupe arylcarbamoyle.

7. Procédé comme revendiqué dans la revendication 6, dans lequel le solvant aprotique est choisi à partir d'un groupe constitué par du N,N-diméthylformamide, du N,N-diméthylacétamide, de la N-méthylpyrrolidone, et de la N,N'-diméthylimidazolidone.

8. Procédé comme revendiqué dans la revendication 6, dans lequel le solvant polaire aprotique est utilisé en une quantité de 2 à 10 fois la quantité du composé représenté par la formule (III).

9. Procédé comme revendiqué dans la revendication 6, dans lequel un rapport molaire de t-butoxyde de sodium sur le composé représenté par la formule (III) se situe dans la gamme de 1 à 10.

10. Procédé comme revendiqué dans la revendication 6, comprenant des étapes de chauffer et d'agiter le composé représenté par la formule (III), sous une atmosphère d'azote, dans le solvant aprotique, en présence de t-butoxyde de sodium ; d'élever une température ; et ensuite de faire réagir le composé avec du dioxyde de carbone.
